# EUROPEAN PATENT APPLICATION

(11) **EP 4 190 902 A1**
(43) Date of publication of application: **07.06.2023**
(21) Application number: 21848897.1
(22) Date of filing: 30.07.2021
(51) Int. Cl.: C12N 15/50, A61K 35/76, A61K 39/215, A61K 48/00, A61P 31/14, C12N 7/01, C12N 15/863

(54) **RECOMBINANT VACCINIA VIRUS**

(30) Priority: 31.07.2020 JP 2020130717; 24.12.2020 JP 2020215454; 06.05.2021 JP 2021078781
(71) Applicant: Tokyo Metropolitan Institute of Medical Science, Tokyo 156-8506 (JP)
(72) Inventor: KOHARA, Michinori, Tokyo 156-8506 (JP); YASUI, Fumihiko, Tokyo 156-8506 (JP); ITOH, Yasushi, Ohtsu-shi, Shiga 520-2192 (JP); ISHII, Koji, Tokyo 162-8640 (JP)
(74) Representative: J A Kemp LLP
(86) International application number: PCT/JP2021/029240
(87) International publication number: WO 2022/025298

(57) **Abstract**

The present invention provides a recombinant vaccinia virus serving as a clinically usable preventive vaccine for COVID-19 (vaccine for SARS-CoV-2), etc.

The recombinant vaccinia virus of the present invention is characterized by comprising the whole or part of cDNA encoding nonstructural proteins or structural protein derived from SARS-CoV-2, and an expression promoter.

## Description

### Technical Field

The present invention relates to a recombinant vaccinia virus serving as a vaccine for SARS-CoV-2, etc.

### Background Art

Highly pathogenic novel coronavirus (SARS-CoV-2)-caused infectious disease (COVID-19), which is regarded as having been first identified in 2019 in Wuhan, China, is then raging in 2020 in all the countries of the world. According to verifiable sources, the number of infected people as of July 2020 exceeds 16 million all over the world, and also exceeds 30,000 in Japan. Among them, the number of deaths exceeds 650,000 all over the world, and has reached about 1,000 in Japan.

Meanwhile, about 30% of people recovered from COVID-19 show insufficient immune induction even after viral clearance (Non-patent Document 1); and hence there is a concern about the risk of re-infection with SARS-CoV-2. On the other hand, immunity induced by infection with common cold coronaviruses will reduce or disappear within a relatively short period of time (e.g., one or two years after infection), so that infection is repeated periodically. Thus, many human beings have no immunity against SARS-CoV-2 under the present circumstances, even patients who have been infected once will experience a reduction in their immunity within a short period of time, and COVID-19 may further continue to spread throughout the world. In view of these facts, it is essential to develop a preventive vaccine for COVID-19 (vaccine for SARS-CoV-2), which is capable of strongly inducing immunity and maintaining the immunity for a long period of time.

Moreover, gene mutations may occur in SARS-CoV-2 as a result of continuous spreading; and hence there is a demand for a vaccine with wide-ranging cross-reactivity which is also applicable to mutation-induced changes in antigenicity. Further, since the appearance of SARS coronavirus (CoV) in 2002, novel types of human-infecting CoV (e.g., MERS-CoV, SARS-CoV-2 found this time) have appeared within a short period of time, so that there is a possibility that unknown types of CoV will further appear in the future. Thus, it is also an important problem to develop a universal preventive vaccine for CoV infectious disease, which exerts a protective effect against multiple types of CoV

### Prior Art Documents

Non-patent Document 1: MedRxiv, 2020 (Fan Wu et al., Neutralizing antibody responses to SARS-CoV-2 in a COVID-19 recovered patient cohort and their implications., medRxiv preprint doi: https://doi.org/10.1101/2020.03.30.20047365.)

### Summary of the Invention

Under these circumstances, there has been a demand for the development of a clinically usable preventive vaccine for COVID-19 (vaccine for SARS-CoV-2), etc.

The present invention has been made in consideration of the above situation, and provides a recombinant vaccinia virus and a pharmaceutical composition, etc., as shown below.
(1) A recombinant vaccinia virus, which comprises the whole or part of cDNA encoding nonstructural proteins or structural proteins derived from SARS-CoV-2, and an expression promoter.
(2) The recombinant vaccinia virus according to (1) above, wherein the vaccinia virus is of the strain DIs.
(3) The recombinant vaccinia virus according to (1) or (2) above, wherein the cDNA encoding nonstructural proteins comprise cDNA encoding the ORF1b region (a nonstructural protein gene region encoding proteins essential for virus replication) in the nonstructural proteins derived from SARS-CoV-2.
(4) The recombinant vaccinia virus according to any one of (1) to (3) above, wherein the cDNA encoding nonstructural proteins is any of DNAs shown in (a) to (d) below:
   (a) DNA consisting of the nucleotide sequence shown in SEQ ID NO: 1;
   (b) DNA sharing 80% or more identity with DNA consisting of a nucleotide sequence complementary to the nucleotide sequence shown in SEQ ID NO: 1, and encoding nonstructural proteins derived from SARS-CoV-2;
   (c) DNA consisting of the nucleotide sequence shown in SEQ ID NO: 3; and
   (d) DNA sharing 80% or more identity with DNA consisting of a nucleotide sequence complementary to the nucleotide sequence shown in SEQ ID NO: 3, and encoding nonstructural proteins derived from SARS-CoV-2.
(5) The recombinant vaccinia virus according to (1) or (2) above, wherein the cDNA encoding structural proteins comprise cDNA encoding the spike protein in the structural protein derived from SARS-CoV-2.
(6) The recombinant vaccinia virus according to (1), (2) or (5) above, wherein the cDNA encoding structural proteins is any of DNAs shown in (a) to (d) below:
   (a) DNA consisting of the nucleotide sequence shown in SEQ ID NO: 5;
   (b) DNA sharing 80% or more identity with DNA consisting of a nucleotide sequence complementary to the nucleotide sequence shown in SEQ ID NO: 5, and encoding structural proteins derived from SARS-CoV-2;
   (c) DNA consisting of the nucleotide sequence shown in SEQ ID NO: 7; and
   (d) DNA sharing 80% or more identity with DNA consisting of a nucleotide sequence complementary to the nucleotide sequence shown in SEQ ID NO: 7, and encoding structural proteins derived from SARS-CoV-2.
(7) The recombinant vaccinia virus according to any one of (1) to (6) above, which is capable of retaining stability even under room temperature.
(8) A pharmaceutical composition, which comprises the recombinant vaccinia virus according to any one of (1) to (7) above.
(9) The pharmaceutical composition according to (8) above, which is a prophylactic agent for SARS-CoV-2 infectious disease.
(10) The pharmaceutical composition according to (8) above, which is a therapeutic agent for SARS-CoV-2 infectious disease.
(11) A vaccine for SARS-CoV-2, which comprises the recombinant vaccinia virus according to any one of (1) to (7) above.

### Effects of the Invention

The present invention enables the provision of a recombinant vaccinia virus serving as a clinically usable preventive vaccine for COVID-19 (vaccine for SARS-CoV-2), and a pharmaceutical composition comprising the same, etc. The recombinant vaccinia virus of the present invention serves as a vaccine for SARS-CoV-2 which is capable of strongly inducing immunity and maintaining the immunity for a long period of time, and is very useful in the prevention or treatment of COVID-19 infectious disease. Moreover, the recombinant vaccinia virus of the present invention is capable of retaining stability as a vaccine for SARS-CoV-2 even under room temperature, and is excellent in practicality because the temperature for its storage and transport may be either refrigeration temperature or room temperature.

### Brief Description of the Drawings

Figure 1 shows the onset protective effect of a SARS-CoV-S gene recombinant vaccine (recombinant vaccinia virus) (the actual results obtained with a SARS vaccine).
Figure 2 shows gene sequence homology among coronavirus genomes.
Figure 3 shows the results of SARS-CoV-S gene recombinant vaccine (rVV-S) inoculation test on rabbits (vaccinia virus LC16m8 pre-immunized group and non-inoculated group).
Figure 4 shows the genetic constitution of a SARS-CoV-2 gene recombinant vaccinia virus.
Figure 5 shows the outline of SARS-CoV-2 gene recombinant vaccines.
Figure 6 shows the constitution of pSMART-DIs-L3-mnCoV-Japan-1b GND-GPTF vector.
Figure 7 shows the constitution of pSMART-DIs-L3-mnCoV-Japan-S-GPTF vector.
Figure 8 shows the results identified for SARS-CoV-2 mRNA expression in cells infected with a SARS-CoV-2 gene recombinant vaccinia virus (rDIs-1b-GND), as measured by Real-Time Detection polymerase chain reaction.
Figure 9 shows the results identified for SARS-CoV-2 spike protein expression in cells infected with a SARS-CoV-2 gene recombinant vaccinia virus (rDIs-S), as measured by Western blotting.
Figure 10 shows the protective mechanism of preventive vaccines for novel coronavirus (SARS-CoV-2) (SARS-CoV-2 gene recombinant vaccinia viruses).
Figure 11 shows the results confirmed for the expression of the introduced antigen in the prepared recombinant vaccines (rDIs-S and rDIs-1b-GND), as measured by Western blotting.
   A (left panel) shows the results of S protein detection with an antibody specific to the SARS-CoV-2 spike protein (S protein).
   B (right panel) shows the results of 1b protein detection with an antibody specific to a SARS-CoV-2 ORF1b constituent protein (nonstructural proteins encoded by the ORF1b region).

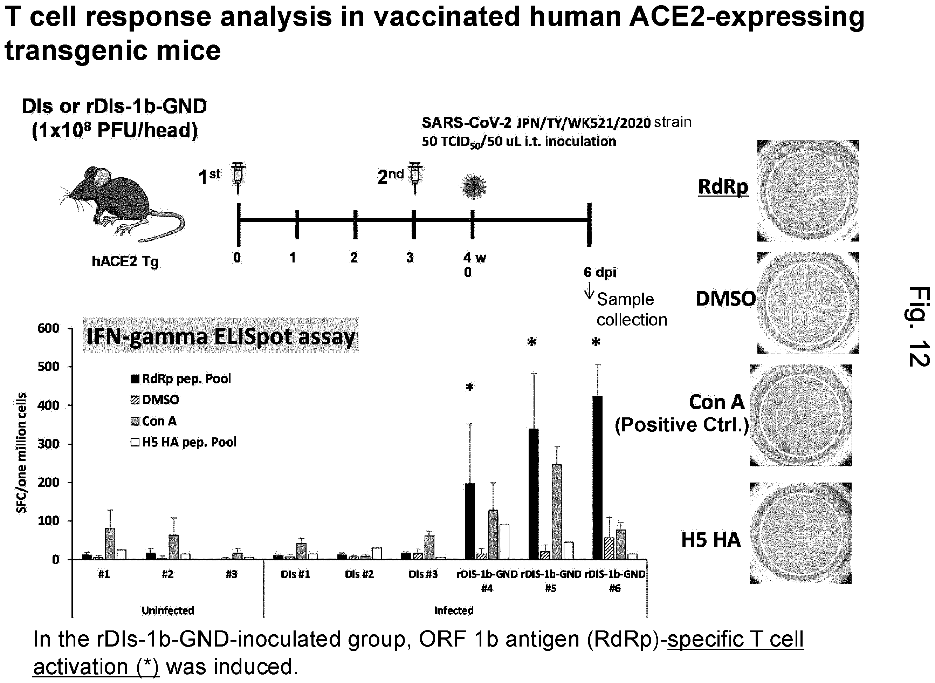

Figure 12 shows the results analyzed for SARS-CoV-2-specific T cell responses in rDIs-1b-GND-inoculated human ACE2-expressing transgenic mice (hACE2-expressing Tg mice).

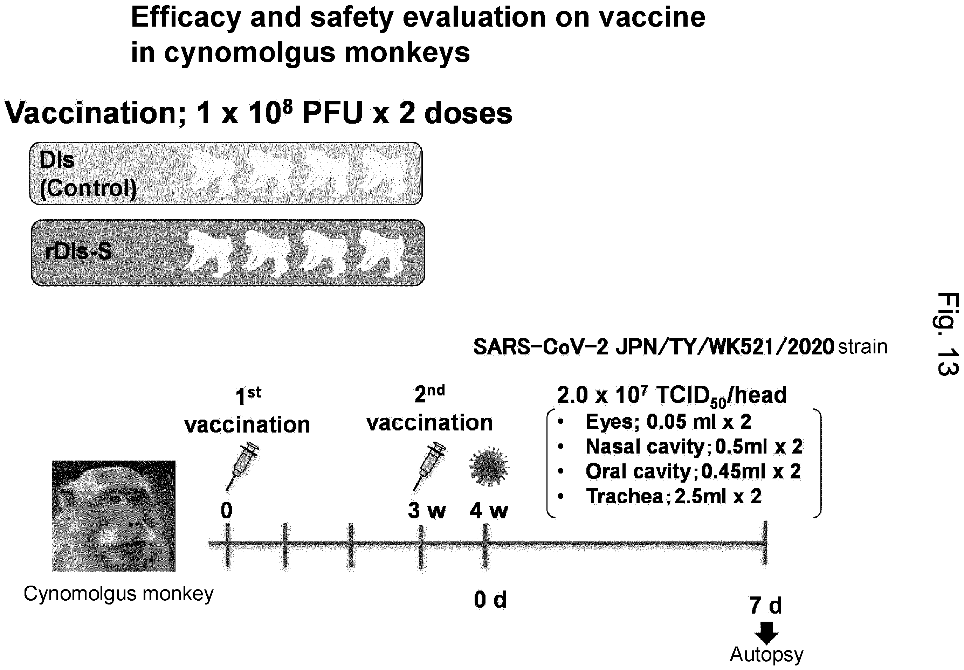

Figure 13 shows how to evaluate the efficacy and safety of the recombinant vaccine (rDIs-S) in cynomolgus monkeys.

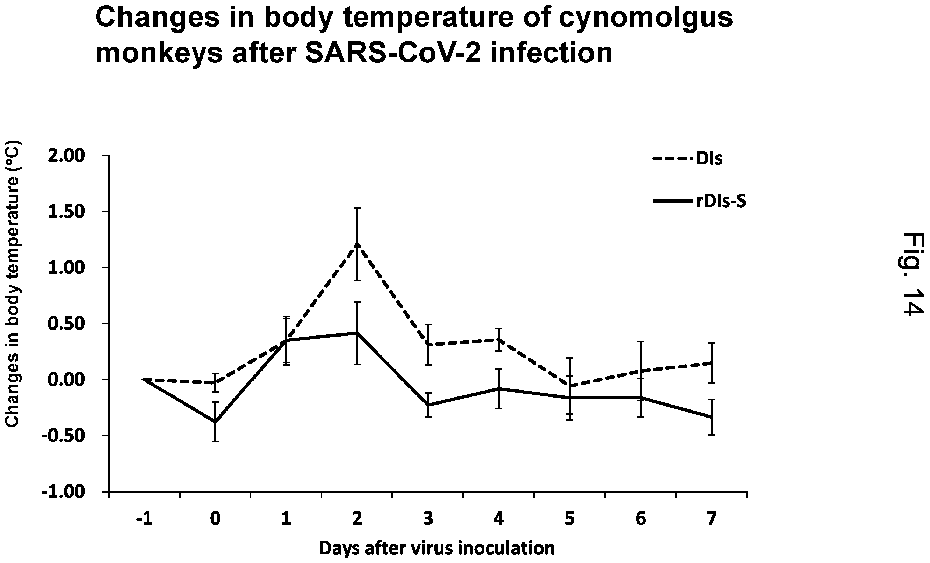

Figure 14 shows the results measured for changes in body temperature after SARS-CoV-2 infection in rDIs-S-inoculated cynomolgus monkeys.

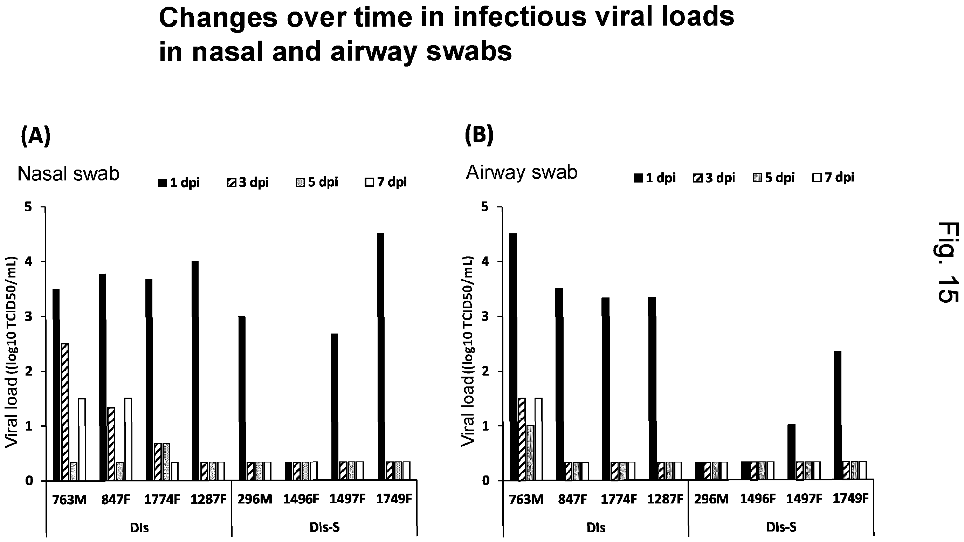

Figure 15 shows the results of changes over time in infectious viral loads in nasal and airway swabs from rDIs-S-inoculated cynomolgus monkeys.
   A (left panel) shows the viral load in nasal swabs, and B (right panel) shows the viral load in airway swabs.

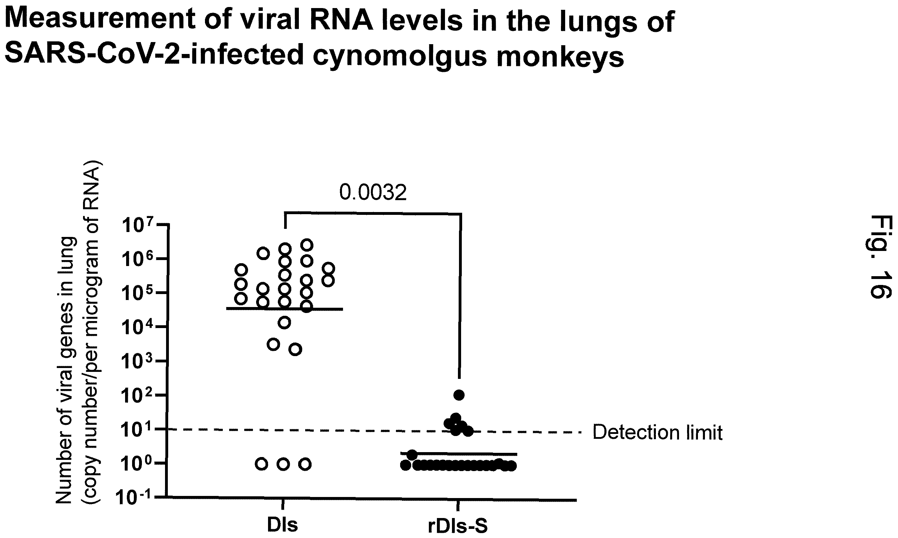

Figure 16 shows the results measured for viral RNA levels in the lungs of rDIs-S-inoculated cynomolgus monkeys at 7 days after SARS-CoV-2 infection.

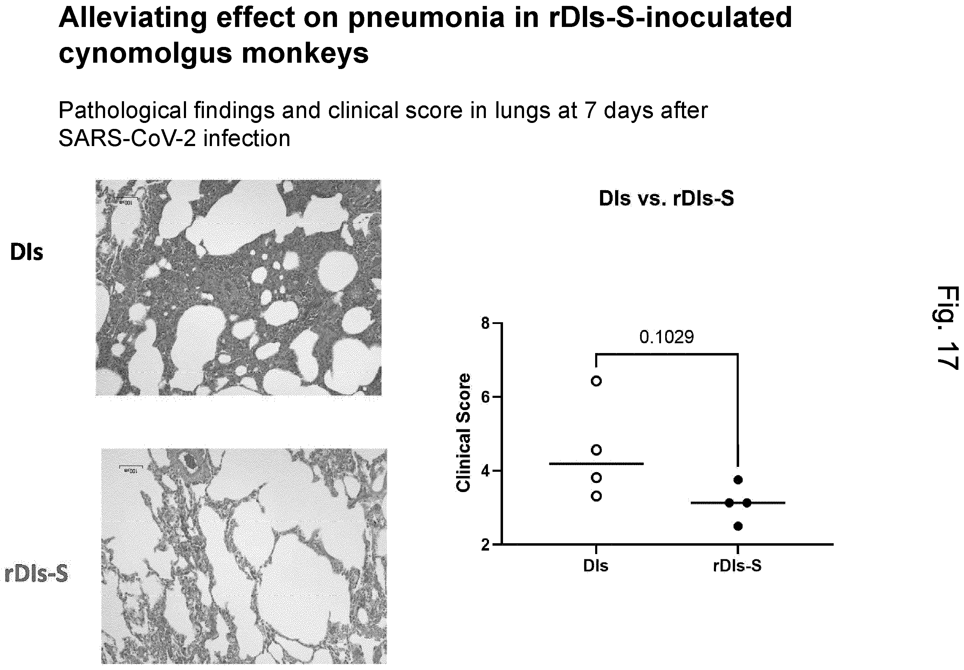

Figure 17 shows the pathological findings (Figure 17, left panel) and clinical score (Figure 17, right panel) in the lungs of rDIs-S-inoculated cynomolgus monkeys at 7 days after SARS-CoV-2 infection.

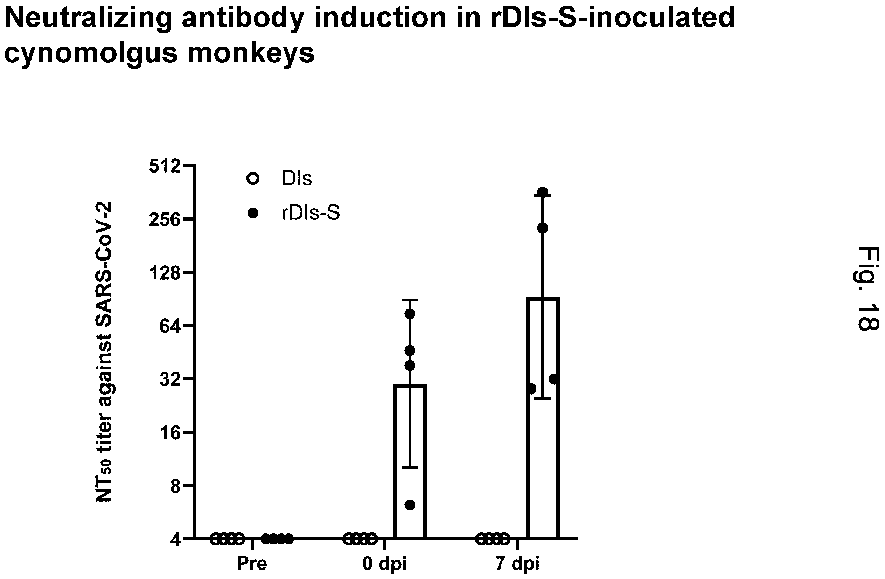

Figure 18 shows the effect of neutralizing antibody induction in rDIs-S-inoculated cynomolgus monkeys.

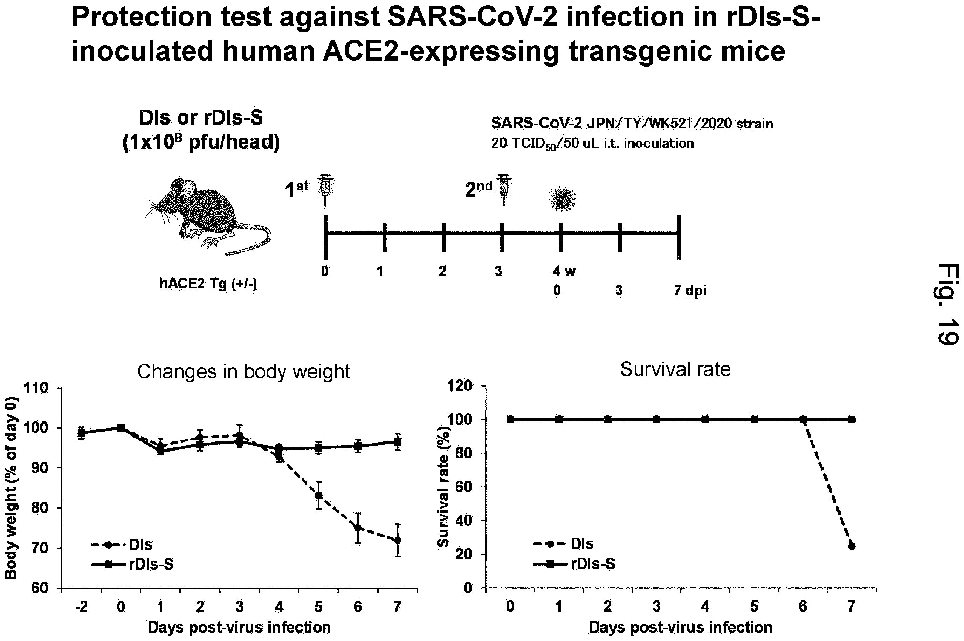

Figure 19 shows the results of protection test against SARS-CoV-2 infection in rDIs-S-inoculated hACE2-expressing Tg mice.

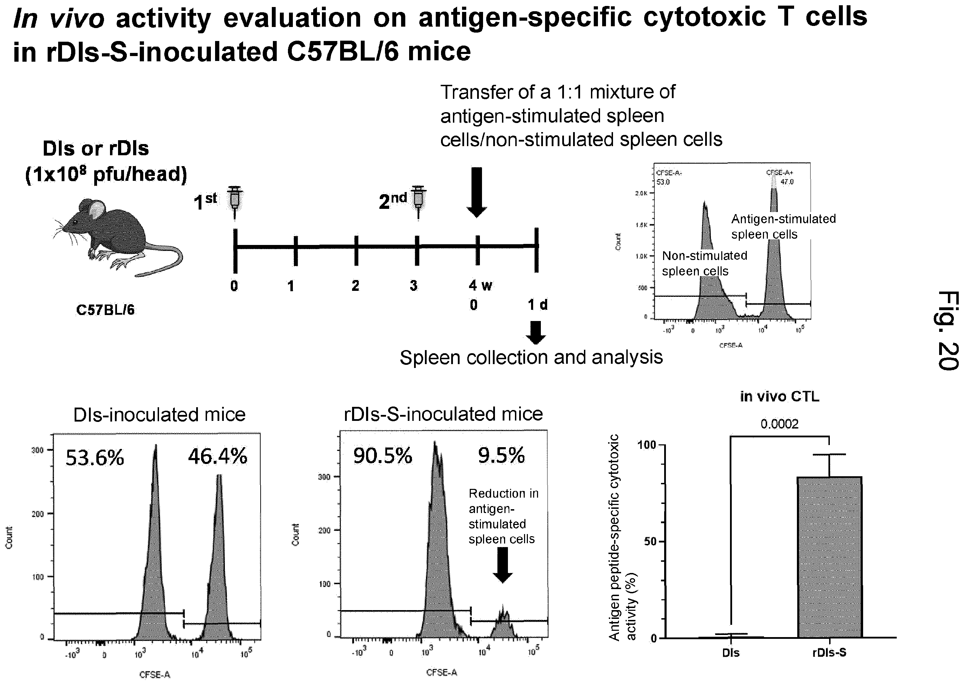

Figure 20 shows the results evaluated for the *in vivo* activity of antigen-specific cytotoxic T cells in rDIs-S-inoculated C57BL/6 mice.

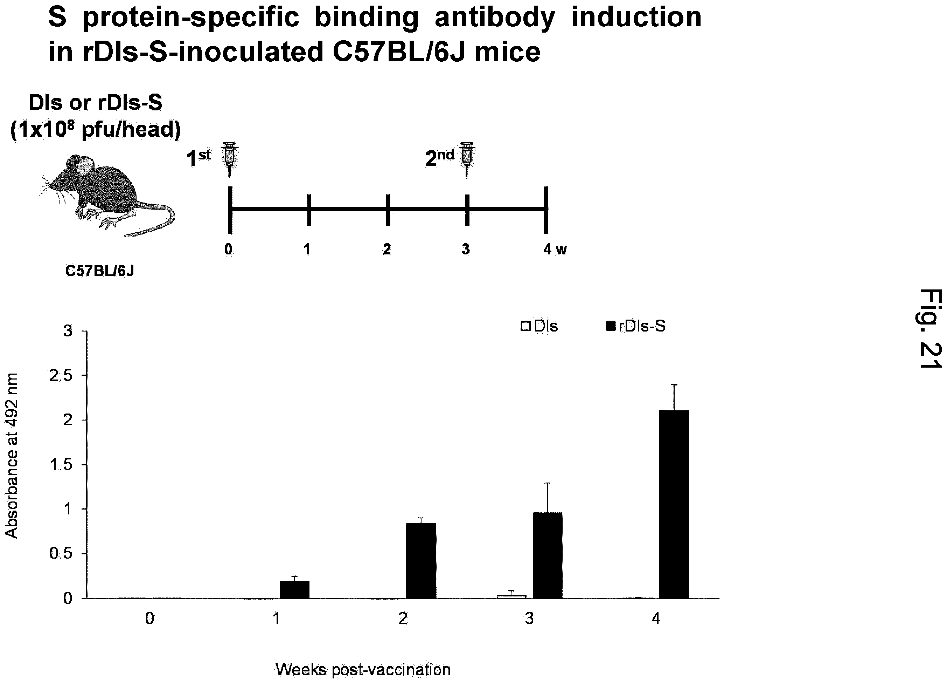

Figure 21 shows the effect of S protein-specific binding antibody induction in rDIs-S-inoculated C57BL/6J mice.

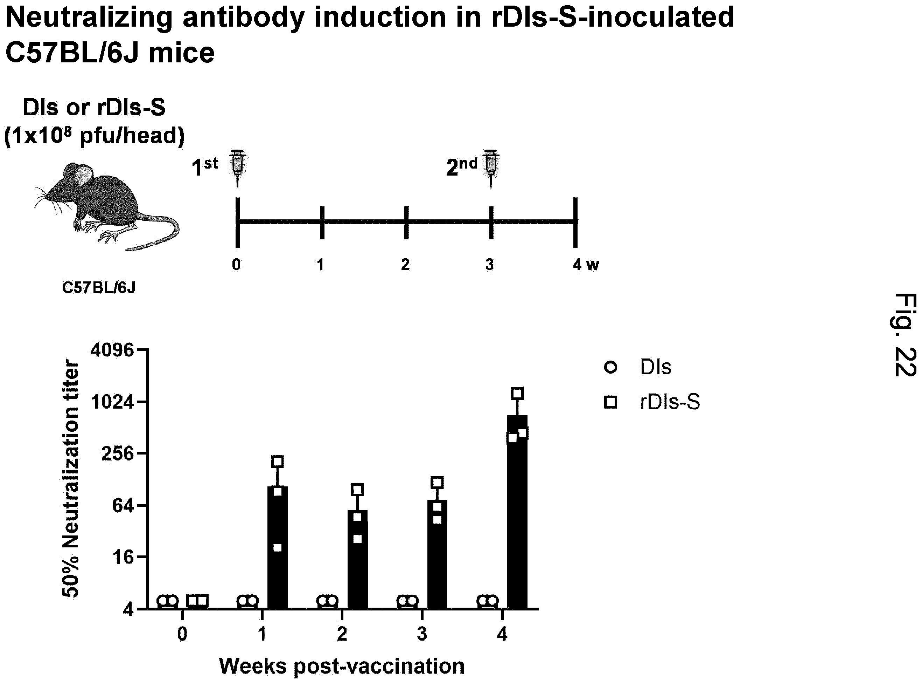

Figure 22 shows the effect of neutralizing antibody induction in rDIs-S-inoculated C57BL/6J mice.

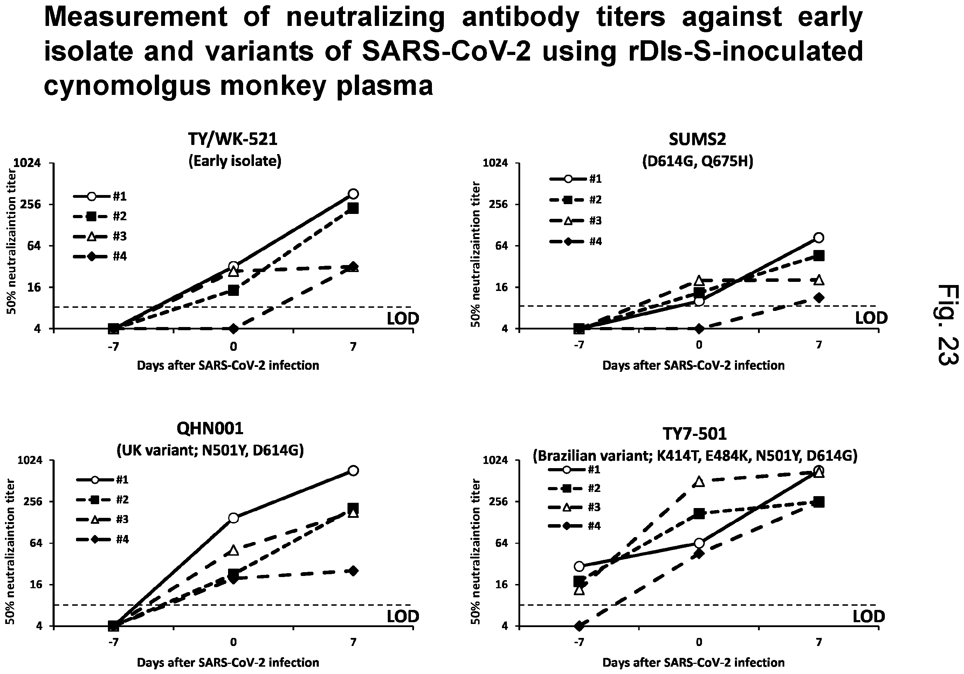

Figure 23 shows the results measured for neutralizing antibody titers against an early isolate of SARS-CoV-2 (TY/WK-521) and variants of SARS-CoV-2 (SUMS2: the variant isolated in the Shiga University of Medical Science; QHN001: the UK variant; TY7-501: the Brazilian variant) using rDIs-S-inoculated cynomolgus monkey plasma. In the graphs, #1 to #4 represent four plasma samples derived from rDIs-S-inoculated cynomolgus monkeys.
   SUMS2 (the variant isolated in the Shiga University of Medical Science) comprises amino acid substitution mutations "D614G, Q675H" in the amino acid sequence (SEQ ID NO: 6) of the SARS-CoV-2 spike protein (S protein),
   QHN001 (the UK variant) comprises amino acid substitution mutations "N501Y, D614G" in the amino acid sequence (SEQ ID NO: 6) of the SARS-CoV-2 S protein, and
   TY7-501 (the Brazilian variant) comprises amino acid substitution mutations "K414T, E484K, N501Y, D614G" in the amino acid sequence (SEQ ID NO: 6) of the SARS-CoV-2 S protein.

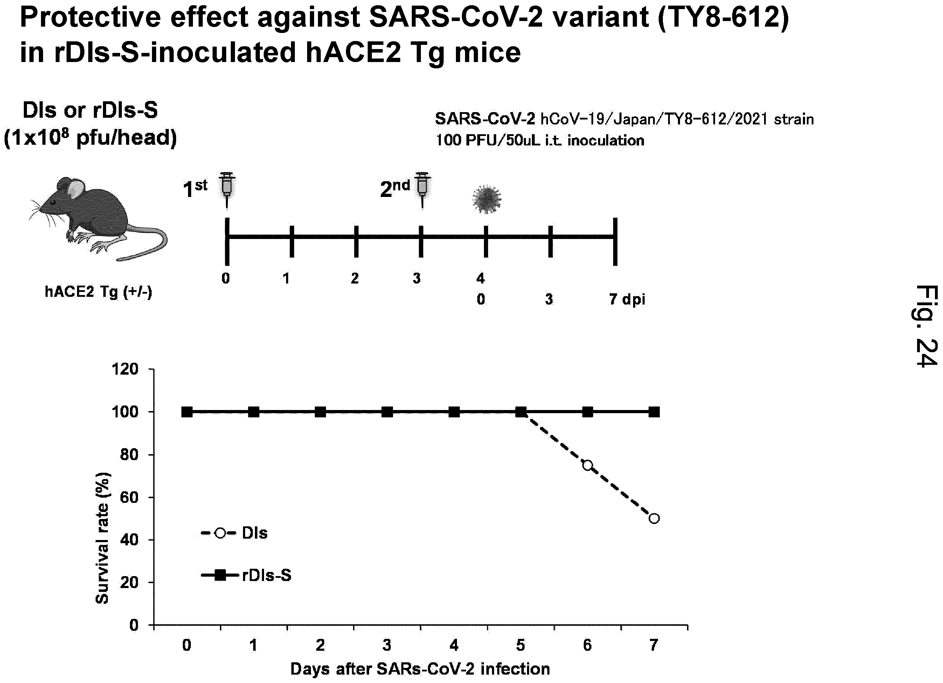

Figure 24 shows the results of a protective effect against a variant of SARS-CoV-2 (TY8-612: the South African variant) in rDIs-S-inoculated hACE2 Tg mice.
   TY8-612 (the South African variant) comprises amino acid substitution mutations "RBD region: K417N, E484K, N501Y; N-terminal region: D80A, D215G; Subdomain 1 region: D614G; S2 region: A701V" and a deletion of amino acids 242 to 244 (Leu-Ala-Leu) in the amino acid sequence (SEQ ID NO: 6) of the SARS-CoV-2 S protein.

### Description of Embodiments

The present invention will be described in more detail below. The scope of the present invention is not limited by the following description, and any embodiments other than those illustrated below may also be carried out with appropriate modifications without departing from the spirit of the invention. It should be noted that this specification incorporates the specification of Japanese Patent Application No. 2020-130717 (filed on July 31, 2020), the specification of Japanese Patent Application No. 2020-215454 (filed on December 24, 2020) and the specification of Japanese Patent Application No. 2021-078781 (filed on May 6, 2021) in their entirety, based on which the present application claims priority. All publications cited herein, including prior art documents, patent gazettes and other patent documents, are incorporated herein by reference.

### 1. Summary of the present invention

The object of the present invention is the development and early practical application of a gene recombinant live vaccine for use as a preventive vaccine against novel coronavirus infectious disease (COVID-19), wherein the gene recombinant live vaccine is designed to comprise a gene derived from SARS-CoV-2 introduced into a highly attenuated strain (DIs) of vaccinia virus serving as a smallpox vaccine. When vaccinia virus is used as a base, immunity against SARS-CoV-2 can be strongly induced within a short period of time after vaccination, and the immunity thus imparted will last over a long period of time, and it can be expected to induce immunity with wide-ranging cross-reactivity even for antigen mutations.

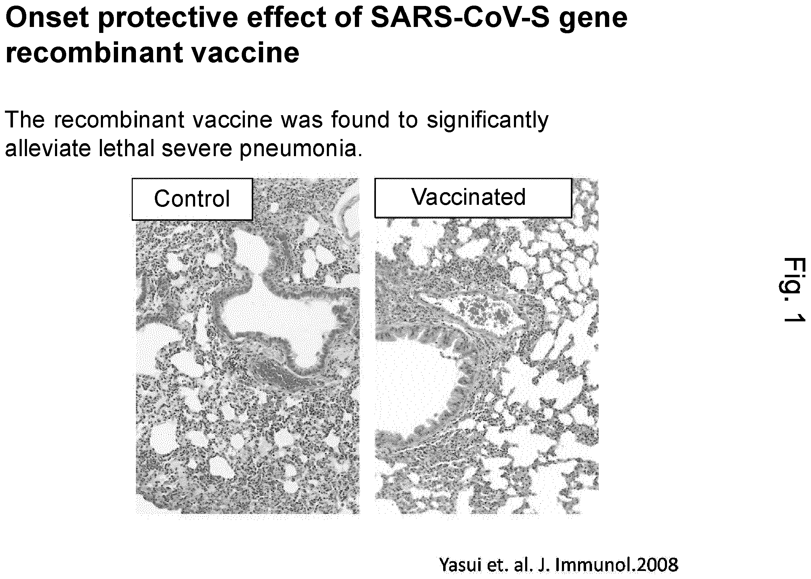

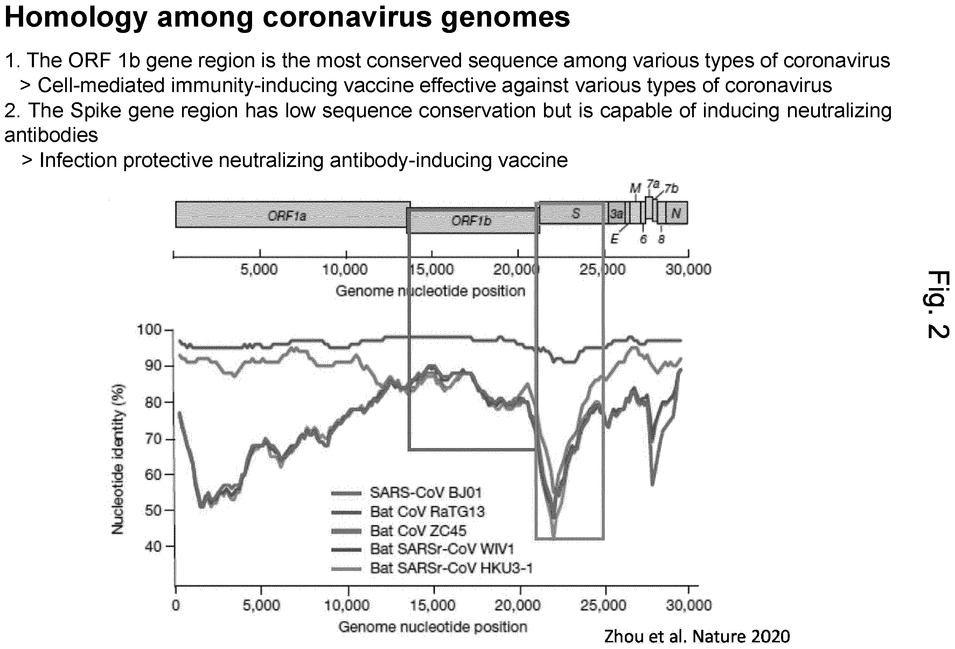

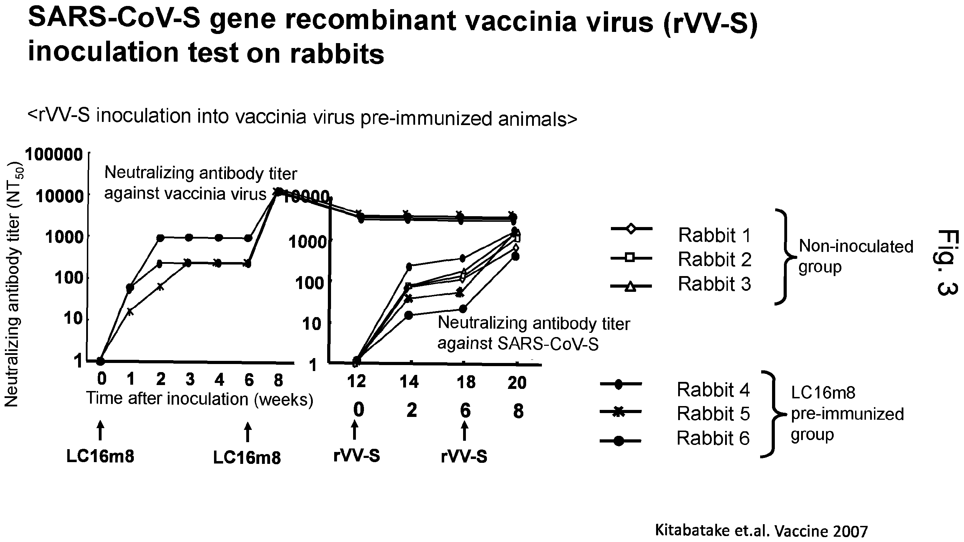

The inventors of the present invention have established the following gene recombinant vaccines as candidates for a preventive vaccine for COVID-19 (vaccine for SARS-CoV-2): 1) a gene recombinant vaccine expressing the spike protein (S protein) which is exposed on the virus particle surface and is important for adsorption and infection to cells (antibody-inducing and T cell-inducing vaccine), on the basis of the actual results obtained with SARS vaccines (Figure 1, Figure 3); and 2) a gene recombinant vaccine expressing a nonstructural protein gene region (ORF1b) which is a region showing the highest gene sequence homology among coronavirus genomes (Zhou et al., Nature. 2020 Mar; 579(7798):270-273)) (Figure 2) and encodes proteins essential for virus replication (T cell-inducing vaccine).

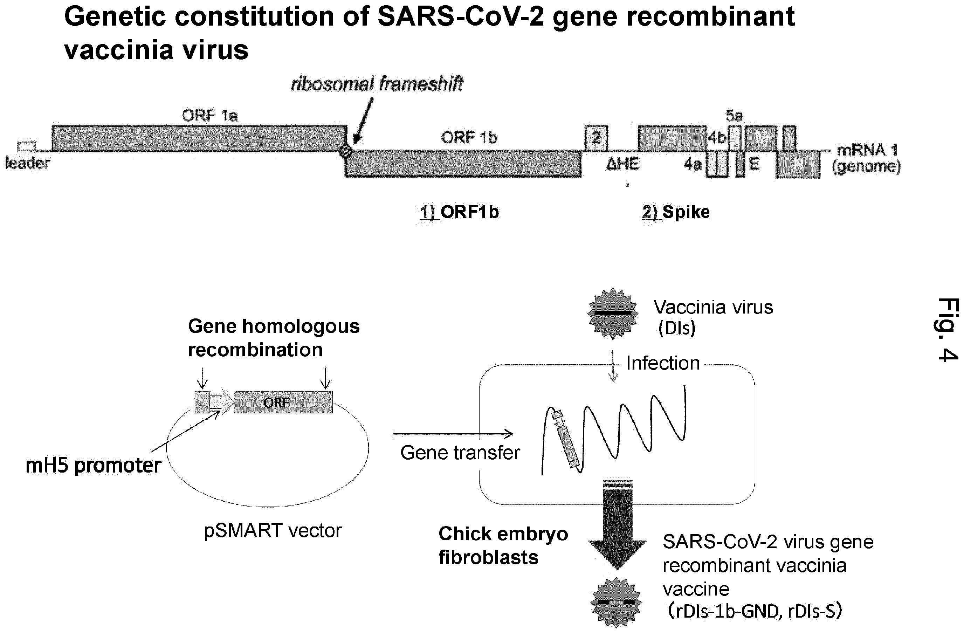

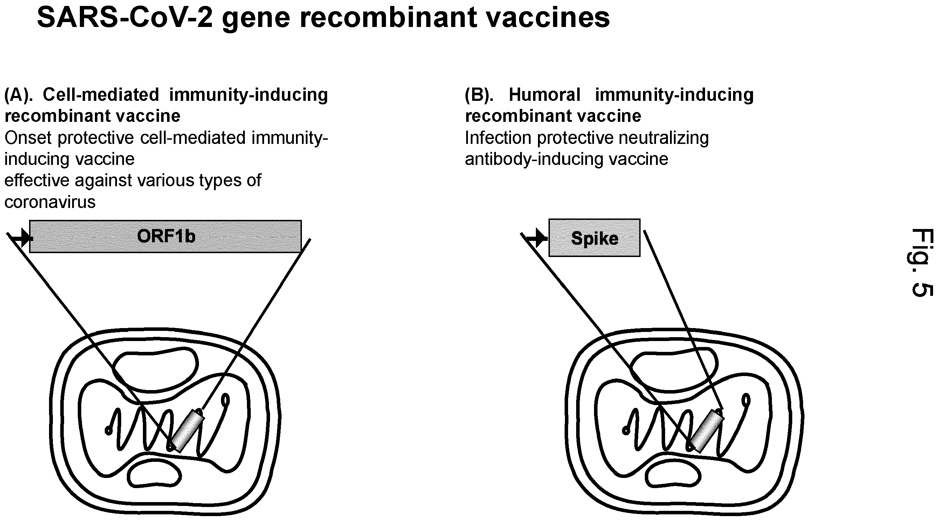

Recombinant vaccines expressing the S protein of SARS-CoV, which have been established and evaluated, were able to induce neutralizing antibodies at 1 week after inoculation, and thereby protect against SARS-CoV challenge in mice (Fumihiko Yasui et al., J Immunol, 181:6337-6348 (2008)) (Figure 1). Moreover, in rabbits which have been inoculated with a smallpox vaccine, such recombinant vaccines were also able to induce SARS-CoV-specific immunity at the same level as in the case of inoculation to non-sensitized rabbits (Masahiro Kitabatake et al., Vaccine, 25: 630-637 (2007)) (Figure 3). Thus, a recombinant vaccinia virus is also deemed to be effective for elderly people who are at high risk of severe COVID-19 and have been inoculated with a smallpox vaccine. Further, with the aim of ensuring higher safety, the inventors of the present invention have also developed a recombinant vaccine based on a highly attenuated vaccinia virus strain, DIs, which is replication-deficient in many normal mammalian cells and was developed by being acclimated in chick embryo fibroblasts (CEFs) by Dr. Isamu Tagaya et al., the National Institute of Health (Isamu Tagaya et al., Nature, 192: 381-382, 1961). SARS-S vaccines based on the strain DIs were also able to protect against SARS-CoV infection (Koji Ishii et al., Virology, 351(2): 368-380, 2006). Moreover, the inventors of the present invention have pursued the development of DIs-based recombinant avian influenza vaccines expressing hemagglutinin (HA) protein genes of the H5 and H7 subtypes (rDIs-H5 HA and rDIs-H7 HA), and have demonstrated that these vaccines exert an onset protective effect within a short period of 1 week after vaccination, and the immunity once imparted shows a long-lasting effect, which may also be regarded as permanent immunity, in a mouse model. On the basis of these findings, the inventors of the present invention have prepared recombinant vaccinia viruses (vaccinia vaccines) capable of expressing SARS-CoV-2 virus structural and nonstructural protein genes (Figures 4 and 5).

### 2. Preparation of recombinant vaccinia virus

As for information about all genes encoding SARS-CoV-2 proteins, genes encoding outer shell protein regions, and genes encoding nonstructural protein regions involved in replication, more specifically, the gene sequence information (SEQ ID NO: 9) of the SARS-CoV-2 virus strain hCoV-19/Japan/AI/I-004/2020 has been registered under GenBank Accession No. LC521925 on the website of the NCBI (https://www.ncbi.nlm.nih.gov/).

Thus, a gene to be contained in the recombinant vaccinia virus of the present invention, i.e., a gene comprising a nonstructural protein region or structural protein region from SARS-CoV-2 can be obtained by commonly used genetic engineering techniques. For example, it is possible to use a nucleic acid synthesis method with a DNA synthesizer, which is commonly used as a genetic engineering technique. Alternatively, it is possible to use a PCR method in which a gene sequence serving as a template is isolated or synthesized, and primers specific to each gene are designed to amplify the gene sequence with PCR equipment, or a gene amplification method in which a cloning vector is used. The above methods can be accomplished by those skilled in the art, in accordance with "Molecular cloning 4th Edt. Cold Spring Harbor Laboratory Press (2012)," etc. For purification of the resulting PCR product, any known method may be used.

In the present invention, a gene (DNA) encoding a nonstructural protein region or structural protein region among all the gene regions of SARS-CoV-2 is used in the preparation of the recombinant vaccinia virus. The nonstructural protein region is a region consisting of ORF1a, ORF1b, ORF3a, ORF6, ORF7a, ORF7b, ORF8 and ORF 10 regions, and for example, the ORF1b region is preferably selected for use in the present invention. Likewise, the structural protein region is a region consisting of spike (S), envelope (E), integral membrane (M) and nucleoprotein (N) regions, and for example, the spike (S) protein region is preferably selected for use in the present invention.

In the present invention, a nucleotide sequence (DNA) encoding the ORF1b region in the nonstructural protein region derived from SARS-CoV-2 is shown in SEQ ID NO: 1, while a nucleotide sequence (DNA) encoding the S protein region in the structural protein region derived from SARS-CoV-2 is shown in SEQ ID NO: 5. Further, mutant DNA of the above nucleotide sequence (DNA) encoding the ORF1b region (modified to substitute other nucleotides for 6 nucleotides in the nucleotide sequence (DNA) encoding the ORF1b region, with the aim of increasing gene expression efficiency within the recombinant vaccinia virus) is shown in SEQ ID NO: 3, while mutant DNA of the above nucleotide sequence (DNA) encoding the S protein region (modified to substitute other nucleotides for 8 nucleotides in the nucleotide sequence (DNA) encoding the S protein region, with the aim of increasing gene expression efficiency within the recombinant vaccinia virus) is shown in SEQ ID NO: 7. However, not only DNAs consisting of the nucleotide sequences shown in SEQ ID NOs: 1, 3, 5 and 7, but also the following DNAs may be used in the present invention.

DNA sharing an identity (homology) of 80% or more, 90% or more, 95% or more, 98% or more or 99% or more with DNA consisting of a nucleotide sequence complementary to the nucleotide sequence shown in SEQ ID NO: 1, and encoding nonstructural proteins derived from SARS-CoV-2 (mutant DNA of the ORF1b region).

DNA sharing an identity (homology) of 80% or more, 90% or more, 95% or more, 98% or more or 99% or more with DNA consisting of a nucleotide sequence complementary to the nucleotide sequence shown in SEQ ID NO: 3, and encoding nonstructural proteins derived from SARS-CoV-2 (mutant DNA of the region comprising the above nucleotide substitution mutations in the ORF1b region).

DNA sharing an identity (homology) of 80% or more, 90% or more, 95% or more, 98% or more or 99% or more with DNA consisting of a nucleotide sequence complementary to the nucleotide sequence shown in SEQ ID NO: 5, and encoding a structural protein derived from SARS-CoV-2 (mutant DNA of the S protein region).

DNA sharing an identity (homology) of 80% or more, 90% or more, 95% or more, 98% or more or 99% or more with DNA consisting of a nucleotide sequence complementary to the nucleotide sequence shown in SEQ ID NO: 7, and encoding a structural protein derived from SARS-CoV-2 (mutant DNA of the region comprising the above nucleotide substitution mutations in the S protein region).

DNA hybridizable under stringent conditions with DNA consisting of a nucleotide sequence complementary to the nucleotide sequence shown in SEQ ID NO: 1, and encoding nonstructural proteins derived from SARS-CoV-2 (mutant DNA of the ORF1b region).

DNA hybridizable under stringent conditions with DNA consisting of a nucleotide sequence complementary to the nucleotide sequence shown in SEQ ID NO: 3, and encoding nonstructural proteins derived from SARS-CoV-2 (mutant DNA of the region comprising the above nucleotide substitution mutations in the ORF1b region).

DNA hybridizable under stringent conditions with DNA consisting of a nucleotide sequence complementary to the nucleotide sequence shown in SEQ ID NO: 5, and encoding a structural protein derived from SARS-CoV-2 (mutant DNA of the S protein region).

DNA hybridizable under stringent conditions with DNA consisting of a nucleotide sequence complementary to the nucleotide sequence shown in SEQ ID NO: 7, and encoding a structural protein derived from SARS-CoV-2 (mutant DNA of the region comprising the above nucleotide substitution mutations in the S protein region).

As used herein, the phrase "encoding nonstructural proteins derived from SARS-CoV-2" is intended to mean encoding a protein that is produced within cells upon virus multiplication. Moreover, a gene encoding the above nonstructural proteins include not only the full-length sequence, but also a partial sequence thereof.

Likewise, the phrase "encoding a structural protein derived from SARS-CoV-2" is intended to mean encoding a protein that forms the outer shell of the virus. Moreover, a gene encoding the above structural protein includes not only the full-length sequence, but also a partial sequence thereof.

The above mutant DNAs may be obtained by chemical synthesis, or may be obtained from cDNA and genomic libraries by known hybridization techniques (e.g., colony hybridization, plaque hybridization, Southern blotting) using DNA consisting of the nucleotide sequence shown in SEQ ID NO: 1, 3, 5 or 7, or a fragment thereof as a probe. Moreover, stringent conditions in the above hybridization include, for example, conditions of 0.1 × SSC to 10 × SSC, 0.1% to 1.0% SDS and 20°C to 80°C, and more detailed conditions include prehybridization at 37°C to 56°C for 30 minutes or longer, and the subsequent one to three washings in 0.1 × SSC, 0.1% SDS at room temperature for 10 to 20 minutes. As to detailed procedures for hybridization, reference may be made to "Molecular cloning 4th Edt. Cold Spring Harbor Laboratory Press (2012)," etc.

The preparation of the recombinant vaccinia virus of the present invention is not limited in any way, and any known techniques may be selected for this purpose. For example, a nucleotide sequence (DNA) encoding a nonstructural protein region (e.g., the ORF1b region) or a structural protein region (e.g., the S protein region) from SARS-CoV-2 may first be inserted into a desired expression vector (plasmid) (e.g., a homologous recombination vector (plasmid) for the strain DIs (Koji Ishii et al. Virology 2006)). The plasmid vector may then be transfected into cells infected with a desired vaccinia virus strain (e.g., the attenuated vaccinia virus strain DIs) to thereby cause homologous recombination within the vaccinia virus genome, thus preparing a recombinant vaccinia virus expressing the desired region in the nonstructural proteins or structural protein from SARS-CoV-2. Such an expression vector is not limited in any way, and a pSMART^{®} vector carrying a gene sequence region homologous to the strain DIs may be used, by way of example. As an expression promoter to be contained in the recombinant vaccinia virus of the present invention, various expression promoters (e.g., mH5 promoter) previously used for vaccinia virus gene expression may be used for this purpose.

It should be noted that the above attenuated vaccinia virus strain DIs is a highly attenuated mutant deleted for host range genes, which has been established by one-day egg passage from the Dalian strain (DIE) serving as a smallpox vaccine, and this virus strain is capable of multiplication only in Chick Embryo Fibroblast (CEF) cells and was developed by Dr. Isamu Tagaya, the National Institute of Health (later renamed as the National Institute of Infectious Diseases) (Tagaya et al. Nature, 192: 381-382, 1961). Due to large gene deletions, this virus strain is incapable of multiplication in most mammalian cells, including mouse, guinea pig, rabbit and human cells. For this reason, this virus strain ensures its safety even when inoculated into immunodeficient and immunosuppressed patients.

The prepared recombinant vaccinia virus may be analyzed by PCR using the virus genome as a template and primers specific to a gene for the nonstructural protein region or structural protein region from SARS-CoV-2 to thereby confirm the gene transfer of the desired nonstructural protein region or structural protein region.

Moreover, the expression of the desired nonstructural proteins or structural protein can be confirmed by Western blotting using animal cells infected with the prepared recombinant vaccinia virus as samples. It should be noted that antibodies for use in Western blotting may be, for example, commercially available antibodies specifically recognizing the desired nonstructural protein region or structural protein region, or IgGs purified with Protein G from antiserum prepared by immunization with a SARS-CoV-2 polypeptide.

The recombinant vaccinia virus of the present invention has high temperature stability and, for example, can retain stability as a vaccine for SARS-CoV-2 even under room temperature (including, but not limited to, the range of 10°C to 40°C, preferably 10°C to 20°C in most cases). Thus, this vaccinia virus and the pharmaceutical composition and so on described later are excellent in practicality and convenience, because the temperature for their storage and transport may be either refrigeration temperature or room temperature.

### 3. Prophylactic or therapeutic pharmaceutical composition for novel coronavirus infectious disease (COVID-19)

The present invention provides a pharmaceutical composition comprising the above recombinant vaccinia virus, more particularly a pharmaceutical composition serving as a prophylactic and therapeutic agent for novel coronavirus (SARS-CoV-2) infectious disease (COVID-19).

The pharmaceutical composition of the present invention may be introduced *in vivo* in every known manner, e.g., by injection via the intramuscular, intraperitoneal, intracutaneous or subcutaneous route, etc., by inhalation via the nasal, oral or pulmonary route, or by administration via the oral route. Further, the recombinant vaccinia virus contained in the pharmaceutical composition of the present invention may be used in combination with an existing antiviral drug (e.g., interferon). Such an embodiment of combined use is not limited in any way, and the recombinant vaccinia virus of the present invention and an existing antiviral drug may be administered simultaneously, or may be introduced *in vivo* in a manner such that either one of them is first administered, and the other is then administered after a certain time has passed.

Moreover, the pharmaceutical composition of the present invention may be used in admixture with known pharmaceutically acceptable carriers (e.g., excipients, extenders, binders, lubricants), buffering agents, isotonizing agents, chelating agents, coloring agents, preservatives, fragrances, flavorings, sweeteners, etc.

The pharmaceutical composition of the present invention may be administered via the oral or parenteral route, depending on its dosage form, e.g., oral dosage forms (e.g., tablets, capsules, powders, granules, pills, solutions, syrups) or parenteral dosage forms (e.g., injections, external preparations, suppositories, eye drops, nose drops). Preferred examples include local injections into the skin, muscle and abdominal cavity, etc.

The dose may be selected as appropriate for the type of active ingredient, the route of administration, the target to be administered, and/or the age, body weight, sex, symptoms and other conditions of a patient. The daily dose of the recombinant vaccinia virus is around 1000 to 1000000000 PFU (plaque forming units), preferably around 100000 to 100000000 PFU for oral administration, and is around 100 to 1000000000 PFU (plaque forming units), preferably around 1000 to 100000000 PFU for parenteral administration. The virus may be administered once a day or may be administered in divided doses.

The recombinant vaccinia virus of the present invention may be used as a prophylactic or therapeutic vaccine for COVID-19, but it is preferable to measure in advance the antibody titer or cell-mediated immune activity as a vaccine.

For example, the antibody titer against the recombinant vaccinia virus of the present invention or its parent strain DIs may be measured as follows: after these virus strains are inoculated into mice, rabbits or other animals, serum is collected over time from each animal to measure the ELISA titer or LIPS (luciferase immunoprecipitation system) titer of the serum against a SARS-CoV-2 protein. This allows confirmation of the presence or absence of SARS-CoV-2 gene expression and immune responses in the inoculated animals. In the serum of the mice inoculated with the recombinant vaccinia virus of the present invention, an increase in the antibody titers against the SARS-CoV-2 protein may be observed from 1 week after inoculation.

Moreover, for measurement of cell-mediated immune activity, for example, the recombinant vaccinia virus of the present invention or its parent strain DIs may be inoculated into mice, and spleen cells may then be separated from the immunized mice, followed by FACS or ELISPOT assay to measure whether CD4-positive and CD8-positive cells specific to a nonstructural protein or structural protein from SARS-CoV-2 are induced and activated. For example, spleen cells derived from BALB/c mice immunized with the recombinant vaccinia virus of the present invention may be co-cultured with target cells expressing the nonstructural protein or structural protein to thereby detect CD4- and CD8-positive T cells activated in an antigen-specific manner.

The recombinant vaccinia virus of the present invention is capable of inducing cell-mediated immunity against SARS-CoV-2, and in particular, this effect is significant in the recombinant vaccinia virus capable of expressing nonstructural proteins (preferably ORF1b) derived from SARS-CoV-2. Moreover, the recombinant vaccinia virus of the present invention is capable of inducing neutralizing antibodies against SARS-CoV-2, and in particular, this effect is significant in the recombinant vaccinia virus capable of expressing a structural protein (preferably S protein) derived from SARS-CoV-2.

Further, the recombinant vaccinia virus (vaccine for SARS-CoV-2) of the present invention can serve as a vaccine with wide-ranging cross-reactivity which is applicable to not only SARS-CoV-2 which is the original direct target of this vaccinia virus, but also mutation-induced changes in the antigenicity of SARS-CoV-2. Namely, in the pharmaceutical composition of the present invention, which is a therapeutic and prophylactic agent for SARS-CoV-2 infectious disease, and the vaccine for SARS-CoV-2 of the present invention, SARS-CoV-2 may also include so-called variants comprising any mutations such as substitution, deletion and/or addition in the nucleotide sequence or amino acid sequence of SARS-CoV-2. Examples of SARS-CoV-2 variants include SUMS2 (the variant isolated in the Shiga University of Medical Science), QHN001 (the UK variant), TY7-501 (the Brazilian variant), TY8-612 (the South African variant) and so on.

Moreover, the recombinant vaccinia virus of the present invention may also be used as a universal preventive vaccine for CoV infectious disease, which also exerts a protective effect against unknown novel types of coronavirus (CoV) that are likely to appear in the future.

The present invention will be further described in more detail by way of the following examples, although the present invention is not limited to these examples.

### [Example 1]

### 1. Method

### (1) Preparation of SARS-CoV-2 virus structural protein gene and nonstructural protein gene recombinant vaccinia vaccines

### (i) Selection of SARS-CoV-2 virus strains for use in gene recombination

On the basis of the gene sequence information of the SARS-CoV-2 virus strain hCoV-19/Japan/AI/I-004/2020 (GenBank Accession No. LC521925) (SEQ ID NO: 9), the following four genes were synthesized.
· Nonstructural protein 1b (ORF1b) gene region nCoV-Japan-1b (8198 bp) (SEQ ID NO: 1)
· Nonstructural protein 1b (ORF1b) gene region mnCoV-Japan-1b GND (8198 bp) (SEQ ID NO: 3)
· Spike protein gene region nCoV-Japan-S (3926 bp) (SEQ ID NO: 5)
· Spike protein gene region mnCoV-Japan-S (3926 bp) (SEQ ID NO: 7)

Among the above four genes, the gene of SEQ ID NO: 1 is the wild-type ORF1b gene, and the gene of SEQ ID NO: 5 is the wild-type spike protein gene.

On the other hand, the genes of SEQ ID NOs: 3 and 7 are mutant genes that have been optimized to improve gene expression efficiency in vaccinia virus on the basis of the genes of SEQ ID NOs: 1 and 5, respectively.

In more detail, the mutant gene of SEQ ID NO: 3 comprises T (thymine) to C (cytosine) substitutions at positions 2445, 2448, 4998, 5001 and 6000, and G (guanine) to A (alanine) substitution at position 2539 (i.e., 6 nucleotide substitutions in total) in the nucleotide sequence shown in SEQ ID NO: 1.

Likewise, the mutant gene of SEQ ID NO: 7 comprises T (thymine) to C (cytosine) substitutions at positions 99, 102, 2364, 2367, 3213, 3216, 3417 and 3420 (i.e., 8 nucleotide substitutions in total) in the nucleotide sequence shown in SEQ ID NO: 5.

Among the above four genes, the genes of SEQ ID NOs: 3 and 7 were used to establish recombinant vaccinia viruses in this example.

### (ii) Cloning of two synthetic genes into pSMART-DIs-L3 vector

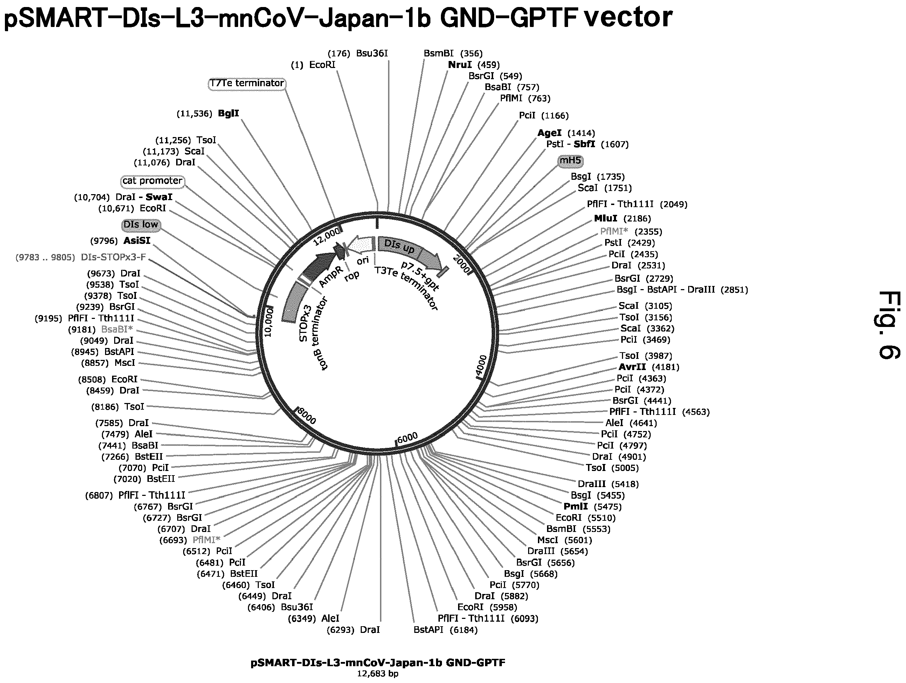

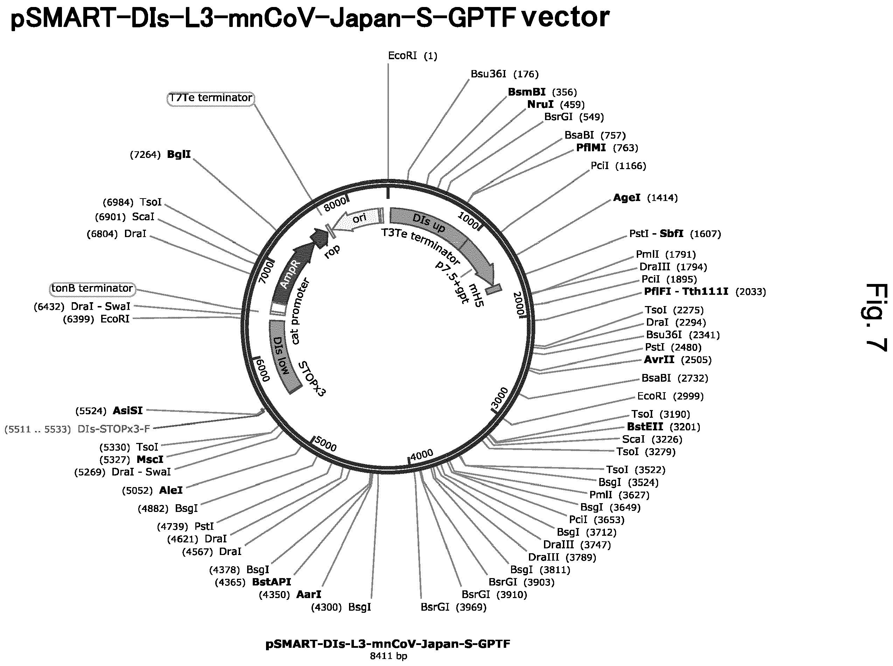

Two synthetic genes (the genes of SEQ ID NOs: 3 and 7) were each inserted into a recombinant vector for DIs recombinant vaccine preparation: pSMART-DIs-L3 (SEQ ID NO: 10) (Figures 6 and 7). These recombinant vectors were used to pursue the establishment of recombinant vaccinia viruses (rDIs-1b-GND and rDIs-S) (Figure 4). "rDIs-1b-GND" denotes a recombinant vaccinia virus obtained by using a vector engineered to carry the gene of SEQ ID NO: 3 (pSMART-DIs-L3-mnCoV-Japan-1b GND-GPTF), and is also referred to as "rDIs-mnCoV1b-GND." "rDIs-S" denotes a recombinant vaccinia virus obtained by using a vector engineered to carry the gene of SEQ ID NO: 7 (pSMART-DIs-L3-mnCoV-Japan-S-GPTF), and is also referred to as "rDIs-mnCoV-S."

### (2) Evaluation of SARS-CoV-2 virus nonstructural protein gene or structural protein gene recombinant vaccinia vaccine

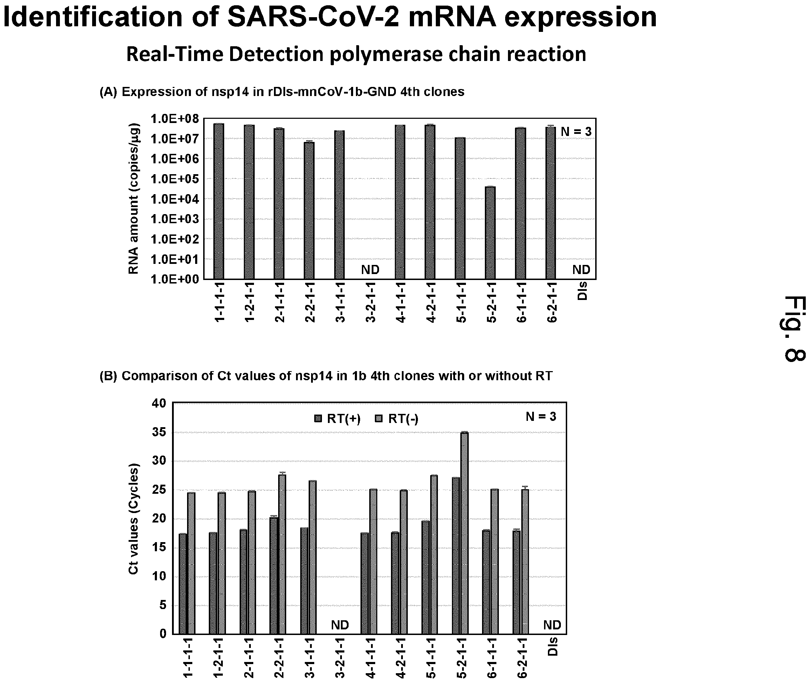

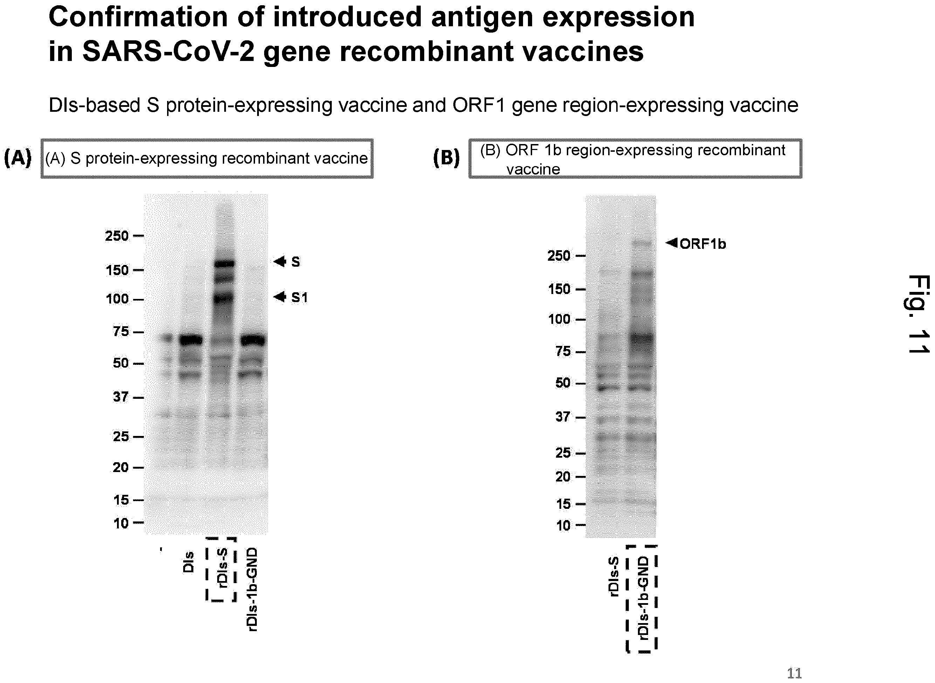

(i) After confirming the gene sequence inserted into the ORF1b nonstructural protein gene recombinant DIs (rDIs-1b-GND), the expression of mRNA was confirmed by Real-Time Detection polymerase chain reaction (RTD-PCR) (Figure 8), and the expression of the ORF1b constituent protein was confirmed by Western blotting (Figure 11B).

The primers and probe, reaction solution composition and reaction conditions used for the above RTD-PCR are as shown below.

### <Primers and probe>

Forward Primer (HKU-ORF1b-nsp14F):
   5'-TGGGGYTTTACRGGTAACCT-3' (SEQ ID NO: 11)
Reverse primer (HKU-ORF1b-nsp14R):
   5'-AACRCGCTTAACAAAGCACTC-3' (SEQ ID NO: 12)
Probe (HKU-ORF1b-nsp141P):
   5'-FAM-TAGTTGTGATGCWATCATGACTAG-TAMRA-3' (SEQ ID NO: 13)

### <Reaction solution composition>

| | |
|---|---|
| Water (RNase free) | 8.5 µL |
| 4x Reaction mix | 5 µL |
| Forward Primer (10 µM) | 1 µL |
| Reverse Primer (10 µM) | 1 µL |
| Probe (10 µM) | 0.5 µL |
| RNA sample | 4 µL |
| Final reaction volume | 20 µL |

### <Reaction conditions>

**Table 1]**

| Temperature | Time | Number of cycles |
|---|---|---|
| 50°C | 5 minutes | 1 |
| 90°C | 20 seconds | |
| 95°C | 5 seconds | 40 |
| 60°C | 30 seconds | |

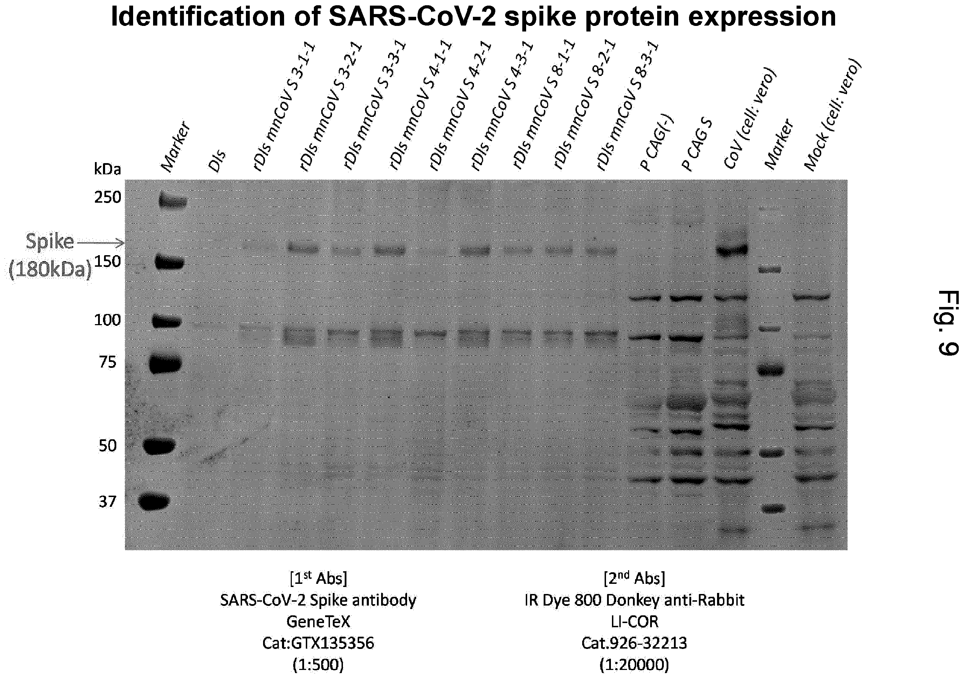

(ii) After confirming the gene sequence inserted into the spike protein gene region recombinant DIs (rDIs-S), the expression of the spike protein was confirmed by Western blotting (Figure 9, Figure 11A).

### 2. Results

(1) After confirming the gene sequence inserted into the ORF1b nonstructural protein gene recombinant DIs (rDIs-1b-GND), the expression of mRNA was confirmed by Real-Time Detection polymerase chain reaction (RTD-PCR) (Figure 8). Good mRNA expression was confirmed in 10 of 12 clones. Moreover, the expression of the ORF1b constituent protein in rDIs-1b-GND was confirmed by Western blotting (Figure 11B).
(2) After confirming the gene sequence inserted into the spike protein gene region recombinant DIs (rDIs-S), the expression of the spike protein was confirmed by Western blotting (Figure 9, Figure 11A). Good spike protein expression was confirmed in 9 of 9 clones.

### 3. Discussion

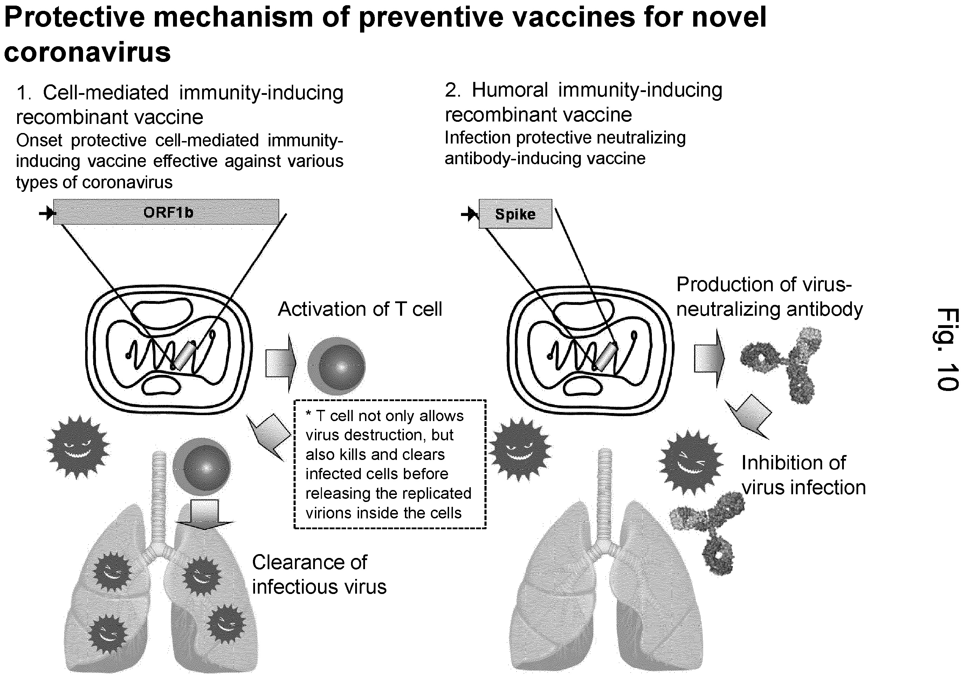

The vaccine (rDIs-1b-GND) against the ORF1b nonstructural protein derived from SARS-CoV-2 can serve as a preventive vaccine effective in inducing cell-mediated immunity. Likewise, the vaccine (rDIs-S) against the spike protein derived from SARS-CoV-2 can serve as a preventive vaccine effective in inducing neutralizing antibodies (Figure 10).

### [Example 2]

rDIs-1b-GND was inoculated into human ACE2-expressing transgenic mice (hACE2-expressing Tg mice) to analyze SARS-CoV-2-specific T cell responses in these mice. In more detail, as shown in Figure 12, the mice were inoculated twice with rDIs-1b-GND or DIs (control) at an interval of 3 weeks and, after 1 week, were challenged with SARS-CoV-2, followed by interferon γ ELISpot assay to analyze the activation of antigen-specific T cells against SARS-CoV-2 at 6 days after infection.

As a result, the rDIs-1b-GND-inoculated group showed the activation (i.e., interferon γ production) of SARS-CoV-2-specific T cells (ORF1b antigen (RdRp)-specific T cells) (Figure 12).

### [Example 3]

rDIs-S was evaluated for its efficacy and safety by using cynomolgus monkeys. In more detail, as shown in Figure 13, the cynomolgus monkeys were intracutaneously inoculated twice with rDIs-S or DIs (control) at an interval of 3 weeks and, after 1 week, were challenged with SARS-CoV-2, followed by autopsy at 7 days after infection. The details of the evaluation and the results obtained are explained in (1) to (5) below.
(1) Changes in body temperature after SARS-CoV-2 infection were measured. The control (DIs) group (broken line) showed a 1°C or higher increase in the body temperature at 2 days after SARS-CoV-2 infection, whereas the rDIs-S-inoculated group (solid line) showed no increase in the body temperature (Figure 14).
(2) Changes over time in infectious viral loads in nasal and airway swabs were measured.
   Viral load in nasal swabs: In the DIs-inoculated group, infectious virus was detected until 7 days after infection. In the rDIs-S-inoculated group, infectious virus was detected only at 1 day after infection, and then rapidly cleared. In one of 4 monkeys in the rDIs-S-inoculated group, infectious virus was below the detection limit even at 1 day after infection (Figure 15A).
   Viral load in airway swabs: In the DIs-inoculated group, infectious virus was detected until 7 days after infection in one of 4 monkeys. In the rDIs-S-inoculated group, infectious virus in two of 4 monkeys was below the detection limit even at 1 day after infection, while even two monkeys in which infectious virus was detected showed significantly low values (Figure 15B).
(3) Viral RNA levels in the lungs were measured at 7 days after SARS-CoV-2 infection. In the DIs-inoculated group, high viral RNA levels were detected in almost all the pulmonary lobes of 4 monkeys. In the rDIs-S-inoculated group, viral RNA levels in pulmonary lobes were mostly below the detection limit, thus indicating a good clearance effect (Figure 16).
(4) Pathological findings and clinical score were confirmed in the lungs at 7 days after SARS-CoV-2 infection. In the DIs-inoculated group, an image of pneumonia with interstitial hypertrophy was observed. On the other hand, the rDIs-S-inoculated group showed almost no lesions, thus indicating the ability to alleviate pneumonia (Figure 17).
(5) The effect of neutralizing antibody induction (NT₅₀ titer) was confirmed in the rDIs-S-inoculated group. In the rDIs-S-inoculated group, neutralizing antibodies were detected in all 4 monkeys (0 dpi), and a rapid increase in antibody titers was observed upon SARS-CoV-2 challenge (7 dpi) (Figure 18).

### [Example 4]

rDIs-S was evaluated for its efficacy by using human ACE2-expressing transgenic mice (hACE2-expressing Tg mice) and C57BL/6 mice. The details of the evaluation and the results obtained are explained in (1) and (2) below.

### (1) Protection test against SARS-CoV-2 infection in rDIs-S-inoculated hACE2-expressing Tg mice

As shown in Figure 19, hACE2-expressing Tg mice were inoculated twice with rDIs-S or DIs (control) at an interval of 3 weeks and, after 1 week, were subjected to a challenge experiment with SARS-CoV-2. The DIs-inoculated animals were dead due to sudden changes in their body weight, whereas the rDIs-S-inoculated animals showed little reduction in their body weight, thus resulting in a 100% survival rate (Figure 19).

### (2) Evaluation of antigen-specific cytotoxic T cells for their in vivo activity in rDIs-S-inoculated C57BL/6 mice

As shown in Figure 20, C57BL/6 mice were inoculated twice with rDIs-S or DIs (control) at an interval of 3 weeks and, after 1 week, were intravenously transferred with a 1:1 mixture of spleen cells stimulated with the SARS-CoV-2 S protein and non-stimulated spleen cells. On the following day, their spleens were collected, and the clearance effect on antigen-stimulated cells in the cell-transferred animals was analyzed by flow cytometry. The DIs-inoculated animals were unable to clear S protein peptide-stimulated cells, whereas the rDIs-S-inoculated animals were able to rapidly clear S protein-derived peptide-stimulated cells (Figure 20). This confirmed that rDIs-S caused the induction of cytotoxic T cells specific to the SARS-CoV-2 S protein.

### [Example 5]

rDIs-S was evaluated for its ability to induce antibodies by using C57BL/6J mice. The details of the evaluation and the results obtained are explained in (1) and (2) below.
(1) As shown in Figure 21, C57BL/6J mice were inoculated twice with rDIs-S or DIs (control) at an interval of 3 weeks, and blood was collected weekly before inoculation until 4 weeks after inoculation. Serum samples were diluted 100-fold with a diluent (PBS(-) containing 1% BSA, 0.5% Tween 20 and 2.5 mM EDTA), and then subjected to ELISA to measure the production of binding antibodies (immunoglobulin G; IgG) specific to the SARS-CoV-2 S protein. In the rDIs-S-inoculated animals (n = 3), S protein-specific antibodies were detected from 1 week after rDIs-S inoculation. Further, when the mice were boosted with rDIs-S at 3 weeks after the first inoculation, S protein-specific IgG levels contained in serum after 1 week were increased.
(2) Using the same serum samples as used in (1) above, neutralizing antibody titers against SARS-CoV-2 (TY/WK-521: early isolate) were evaluated by plaque reduction neutralization test. In more detail, the serum samples were diluted 10-fold with a culture medium, and further subjected to 4-fold serial dilution. 10-fold, 40-fold, 160-fold, 640-fold, 2560-fold and 10240-fold diluted serum samples (50 µL) were each mixed with 50 PFU/50 µL of the virus solution, and then reacted at 37°C for 1 hour. Subsequently, the serially diluted serum/virus mixed solutions (100 µL) were each inoculated into VeroE6/TMPRSS2 cells susceptible to SARS-CoV-2 infection, and then adsorbed for 1 hour. After the solution was removed, the cells were washed with the medium, and a 0.6% agarose-containing medium was then added thereto, followed by culturing for 48 hours. The number of plaques formed after 48 hours in a serum-free sample was set to 100%, and the reciprocal of the serum dilution factor at which the number of plaques was halved upon serum addition was calculated as a "50% neutralization titer." As shown in Figure 22, rDIs-S-inoculated animals (n = 3) were found to induce neutralizing antibodies from 1 week after rDIs-S inoculation. Moreover, neutralizing antibody titers were also enhanced upon booster at 3 weeks after the first inoculation.

### [Example 6]

Using plasma samples from the cynomolgus monkeys inoculated with rDIs-S in Example 3 (intracutaneously inoculated twice at an interval of 3 weeks), neutralizing antibody titers against an early isolate of SARS-CoV-2 (TY/WK-521) and variants of SARS-CoV-2 (SUMS2: the variant isolated in the Shiga University of Medical Science; QHN001: the UK variant; TY7-501: the Brazilian variant) were measured by TCID50 assay. The plasma samples used in this example were collected at the time of booster at 3 weeks after the first inoculation of rDIs-S (on day -7 in the graphs shown in Figure 23), SARS-CoV-2 challenge (on day 0 in the graphs shown in Figure 23) and autopsy (on day 7 in the graphs shown in Figure 23). As shown in Figure 23, it was indicated that when the vaccine was inoculated twice, neutralizing antibodies were induced against the early isolate and all the variants.

### [Example 7]

hACE2-expressing Tg mice were inoculated twice with rDIs-S or DIs (control) at an interval of 3 weeks, and then infected through the airway with 100 PFU of the South African variant (TY8-612) of SARS-CoV-2 at 1 week after booster. Their survival rate was observed every day, and the mice were subjected to autopsy at 7 days after infection. As shown in Figure 24, two of 4 mice were dead in the DIs-inoculated group (control), whereas all 5 mice survived in the rDIs-S-inoculated group. rDIs-S inoculation was confirmed to also provide an onset protective effect against this variant (TY8-612).

### Relevant Information and Articles

(1) Masahiro Kitabatake, Shingo Inoue, Fumihiko Yasui, Shoji Yokochi, Masaaki Arai, Kouichi Morita, Hisatoshi Shida, Minoru Kidokoro, Fukashi Murai, Mai Quynh Le, Kouji Matsushima and Michinori Kohara. SARS-CoV spike protein recombinant vaccinia virus efficiently induces neutralizing antibodies in spite of pre-immunization with vaccinia virus. Vaccine 25: 630-637 (2007).
(2) Fumihiko Yasui, Chieko Kai, Masahiro Kitabatake, Shingo Inoue, Misako Yoneda, Shoji Yokochi, Ryoichi Kase, Satoshi Sekiguchi, Kouichi Morita, Tsunekazu Hishima, Hidenori Suzuki, Katsuo Karamatsu, Yasuhiro Yasutomi, Hisatoshi Shida, Minoru Kidokoro, Kyosuke Mizuno, Kouji Matsushima, Michinori Kohara. Prior immunization with SARS-CoV nucleocapsid protein causes severe pneumonia in mice infected with SARS-CoV J. Immunology 181(9):6337-48 (2008).
(3) Jin-Won Youn, Yu-Wen Hu, Nancy Tricoche, Wolfram Pfahler, Mohamed Tarek Shata, Marlene Dreux, François-Loic Cosset, Antonella Folgori, Dong-Hun Lee, Betsy Brotman, and Alfred M. Prince. Evidence for Protection against Chronic Hepatitis C Virus Infection in Chimpanzees by Immunization with Replicating Recombinant Vaccinia Virus. JOURNAL OF VIROLOGY, Nov. 2008, 82 (21): 10896-10905. doi: 10.1128/JVI.01179-08
(4) FRANÇOIS HABERSETZER, GÉRALDINE HONNET, CHRISTINE BAIN, MARIANNE MAYNARD-MUET, VINCENT LEROY, JEAN-PIERRE ZARSKI, CYRILLE FERAY, THOMAS F. BAUMERT, JEAN-PIERRE BRONOWICKI, MICHEL DOFFOËL, CHRISTIAN TREPO,DELPHINE AGATHON, MYEW-LING TOH, MARTINE BAUDIN, JEAN-YVES BONNEFOY, JEAN-MARC LIMACHER, and GENEVIÈVE INCHAUSPÉ. A Poxvirus Vaccine Is Safe, Induces T-Cell Responses, and Decreases Viral Load in Patients With Chronic Hepatitis C. GASTROENTEROLOGY 2011;141:890-899. doi:10.1053/j.gastro.2011.06.009
(5). Satoshi Sekiguchi, Kiminori Kimura, Tomoko Chiyo, Takahiro Ohtsuki, Yoshimi Tobita, Yuko Tokunaga, Fumihiko Yasui, Kyoko Tsukiyama-Kohara, Takaji Wakita, Toshiyuki Tanaka, Masayuki Miyasaka, Kyosuke Mizuno, Yukiko Hayashi, Tsunekazu Hishima, Kouji Matsushima and Michinori Kohara. Immunization with a recombinant vaccinia virus that encodes nonstructural proteins of the hepatitis C virus suppresses viral protein levels in mouse liver. PLoS ONE 7(12):e51656 (2012).
(6) Takeshi Wada, Michinori Kohara and Yasuhiro Yasutomi. DNA vaccine expressing the non-structural proteins of hepatitis C virus diminishes the expression of HCV proteins in a mouse model. Vaccine 31(50):5968-74, doi: 10.1016/j.vaccine.2013.10.037 (2013).
(7) Takahiro Ohtsuki, Kiminori Kimura, Yuko Tokunaga, Kyoko Tsukiyama-Kohara, Chise Tateno, Yukiko Hayashi, Tsunekazu Hishima, and Michinori Kohara. M2 macrophages play critical roles in progression of inflammatory liver disease in hepatitis C virus transgenic mice. J. Virology 2015 Oct 14;90(1):300-7. doi: 10.1128/JVI.02293-15.

### Industrial Applicability

The recombinant vaccinia virus of the present invention serving as a preventive vaccine for COVID-19 (vaccine for SARS-CoV-2) and a pharmaceutical composition comprising the same, etc. are very useful in the prevention or treatment of COVID-19 infectious disease as a vaccine for SARS-CoV-2 which is capable of strongly inducing immunity and maintaining the immunity for a long period of time. Moreover, the recombinant vaccinia virus of the present invention is capable of retaining stability as a vaccine for SARS-CoV-2 even under room temperature, and is excellent in practicality because the temperature for its storage and transport may be either refrigeration temperature or room temperature.

### Sequence Listing Free Text

SEQ ID NO: 3: synthetic DNA
SEQ ID NO: 4: synthetic construct
SEQ ID NO: 7: synthetic DNA
SEQ ID NO: 8: synthetic construct
SEQ ID NO: 10: synthetic DNA
SEQ ID NO: 11: synthetic DNA
SEQ ID NO: 12: synthetic DNA
SEQ ID NO: 13: synthetic DNA

## Claims

1. A recombinant vaccinia virus, which comprises the whole or part of cDNA encoding nonstructural proteins or structural protein derived from SARS-CoV-2, and an expression promoter.

2. The recombinant vaccinia virus according to claim 1, wherein the vaccinia virus is of the strain DIs.

3. The recombinant vaccinia virus according to claim 1 or 2, wherein the cDNA encoding nonstructural proteins comprise cDNA encoding the ORF1b region in the nonstructural protein derived from SARS-CoV-2.

4. The recombinant vaccinia virus according to any one of claims 1 to 3, wherein the cDNA encoding nonstructural proteins is any of DNAs shown in (a) to (d) below:
(a) DNA consisting of the nucleotide sequence shown in SEQ ID NO: 1;
(b) DNA sharing 80% or more identity with DNA consisting of a nucleotide sequence complementary to the nucleotide sequence shown in SEQ ID NO: 1, and encoding a nonstructural protein derived from SARS-CoV-2;
(c) DNA consisting of the nucleotide sequence shown in SEQ ID NO: 3; and
(d) DNA sharing 80% or more identity with DNA consisting of a nucleotide sequence complementary to the nucleotide sequence shown in SEQ ID NO: 3, and encoding a nonstructural protein derived from SARS-CoV-2.

5. The recombinant vaccinia virus according to claim 1 or 2, wherein the cDNA encoding a structural protein comprises cDNA encoding the spike protein in the structural protein derived from SARS-CoV-2.

6. The recombinant vaccinia virus according to claim 1, 2 or 5, wherein the cDNA encoding a structural protein is any of DNAs shown in (a) to (d) below:
(a) DNA consisting of the nucleotide sequence shown in SEQ ID NO: 5;
(b) DNA sharing 80% or more identity with DNA consisting of a nucleotide sequence complementary to the nucleotide sequence shown in SEQ ID NO: 5, and encoding a structural protein derived from SARS-CoV-2;
(c) DNA consisting of the nucleotide sequence shown in SEQ ID NO: 7; and
(d) DNA sharing 80% or more identity with DNA consisting of a nucleotide sequence complementary to the nucleotide sequence shown in SEQ ID NO: 7, and encoding a structural protein derived from SARS-CoV-2.

7. The recombinant vaccinia virus according to any one of claims 1 to 6, which is capable of retaining stability even under room temperature.

8. A pharmaceutical composition, which comprises the recombinant vaccinia virus according to any one of claims 1 to 7.

9. The pharmaceutical composition according to claim 8, which is a prophylactic agent for SARS-CoV-2 infectious disease.

10. The pharmaceutical composition according to claim 8, which is a therapeutic agent for SARS-CoV-2 infectious disease.

11. A vaccine for SARS-CoV-2, which comprises the recombinant vaccinia virus according to any one of claims 1 to 7.
